# EUROPÄISCHE PATENTANMELDUNG

(11) **EP 0 810 194 A1**
(43) Veröffentlichungstag der Anmeldung: **03.12.1997**
(21) Anmeldenummer: 97108446.2
(22) Anmeldetag: 26.05.1997
(51) Int. Cl.: C07C 41/28, C07C 43/13, C07C 43/178

(54) **Verfahren zur Herstellung von 3-Oxyalkylpropan-1-olen**

(30) Priorität: 30.05.1996 DE 19621703
(71) Anmelder: HOECHST AKTIENGESELLSCHAFT, 65926 Frankfurt am Main (DE)
(72) Erfinder: Hölderich, Wolfgang, Prof. Dr., 67227 Frankenthal (DE); Paczkowski, Marcus, Dr., 64289 Darmstadt (DE); Heinz, Dieter, 40670 Meerbusch (DE); Kaiser, Thomas, 65779 Kelkheim (DE)

(57) **Zusammenfassung**

Gegenstand der Erfindung ist ein Verfahren zur Herstellung von 3-Oxyalkylpropan-1-olen durch katalytische Hydrierung von 1,3-Dioxanen. Die Katalysatoren enthalten eine hydrieraktive Komponente auf einem aciden Träger.

## Beschreibung

Die vorliegende Erfindung betrifft ein neues Verfahren zur Herstellung von 3-Oxyalkylpropan-1-olen. Ausgangsverbindungen sind leicht zugängliche 1,3-Dioxane, die in Gegenwart von Katalysatoren, die eine hydrieraktive Komponente und einen aciden Träger enthalten, hydriert werden.

3-Oxyalkylpropan-1-ole werden als Lösungsmittel verwendet. Sie haben ferner Bedeutung als Bausteine für die Gewinnung von Estern, die als Weichmacher für Kunststoffe, insbesondere für Polyvinylchlorid, Verwendung finden. Diese Ester zeichnen sich durch hohe Verträglichkeit mit dem Polymeren, physiologische Unbedenklichkeit und geringe Flüchtigkeit aus. Weiterhin werden 3-Oxyalkylpropan-1-ole als Zwischenprodukte für die Synthese biologisch aktiver Verbindungen eingesetzt.

Es ist bekannt, aliphatische Monoether des 2,2-Dimethylpropan-1,3-diols mit C₃- bis C₂₀-Alkoxygruppen aus cyclischen Acetalen des 2,2-Dimethylpropan-1,3-diols mit geradkettigen oder verzweigten, gesättigten oder ungesättigten aliphatischen Aldehyden mit 3 bis 20 Kohlenstoffatomen (d.h. 5,5-Dimethyl-2-alkyl-1,3-dioxanen) durch Hydrierung in Gegenwart von Kupferchromit- oder Nickeloxidkatalysatoren herzustellen (DE 33 28 561 A1). Kupferchromit und Nickeloxid können dabei gegebenenfalls auch auf inerten Trägern eingesetzt werden.

Die Verwendung von Kupferchromit-Katalysatoren ist aus Gründen des Arbeitsschutzes unerwünscht, denn zu ihrer Herstellung geht man von hochtoxischen Chromaten aus. Auch gegen den Einsatz von Nickelkatalystoren bestehen Bedenken, weil Nickel toxisch und cancerogen ist. Überdies bevorzugt man aus wirtschaftlichen Erwägungen Katalysatoren mit möglichst niedrigem Gehalt an hydrieraktivem Metall. Die geringe Metallkonzentration soll jedoch nicht zu Lasten der Katalysatoreffektivität und -selektivität gehen.

Es bestand daher die Aufgabe ein Verfahren bereitzustellen, das die aufgezeigten Nachteile vermeidet, technisch einfach zu realisieren und allgemein anwendbar ist. Es soll überdies selektiv arbeiten, hohe Ausbeuten der Zielprodukte sicherstellen und sowohl in der Gasphase als auch in flüssiger Phase durchführbar sein.

Die Erfindung besteht in einem Verfahren zur Herstellung von 3-Oxyalkyl-propan-1-olen der allgemeinen Formel (I) 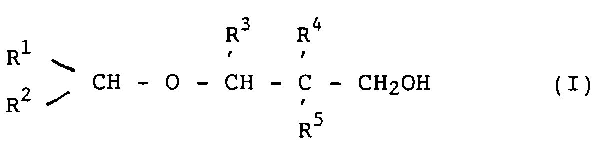 indem man 1,3-Dioxane der allgemeinen Formel (II) 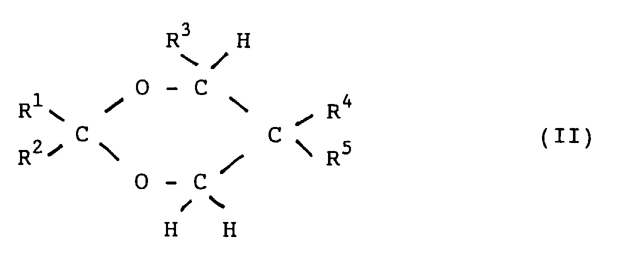 in der R¹, R², R⁴ und R⁵ gleich oder verschieden sind und Wasserstoff, geradkettige oder verzweigte Alkyl-, Alkenyl- oder Alkinylreste mit bis zu 18 Kohlenstoffatomen, Cycloalkyl- oder Cycloalkenylreste mit 5 bis 8 Kohlenstoffatomen, Aryl- Alkylaryl-, Aralkyl-, Aralkenyl- oder Alkenylarylreste mit 6 bis 16 Kohlenstoffatomen oder heterocyclische Reste bedeuten, wobei jeweils die Reste R¹ und R² und/oder R⁴ und R⁵ zusammen mit dem Kohlenstoffatom, an das sie gebunden sind, ein Cycloalkan, Cycloalken oder einen Heterocyclus mit 5 bis 7 Ringgliedern bilden können, R¹, R², R⁴ und R⁵ überdies Substituenten, insbesondere solche, die unter den Reaktionsbedingungen inert sind, tragen können und R³ für Wasserstoff oder einen geradkettigen oder verzweigten Alkylrest steht, bei Drücken von 0,1 bis 35 MPa und Temperaturen von 40 bis 500°C in Gegenwart von Katalysatoren hydriert. Es ist dadurch gekennzeichnet, daß die Katalysatoren als hydrieraktive Komponente ein Metall oder mehrere Metalle der Gruppen VIB, VIIIB und IB des Periodensystems der Elemente, ausgenommen Chrom und Nickel, und einen aciden Träger enthalten.

Bevorzugte 1,3-Dioxane nach der allgemeinen Formel (II) sind Verbindungen, in denen R¹, R², R⁴ und R⁵ gleich oder verschieden sind und Wasserstoff, geradkettige oder verzweigte Alkylreste mit 1 bis 12 und insbesondere 1 bis 6 Kohlenstoffatomen, Alkenyl- oder Alkinylreste mit 2 bis 12 und insbesondere 2 bis 6 Kohlenstoffatomen bedeuten. Bevorzugt sind ferner Verbindungen der allgemeinen Formel (II), in denen R¹, R², R⁴ und R⁵ für Cycloalkyl- oder Cycloalkenylreste mit 5 oder 6 Kohlenstoffatomen, für Aryl-, Alkylaryl-, Aralkyl-, Aralkenyl- oder Alkenylarylreste mit 6 bis 12 Kohlenstoffatomen und für heterocyclische Reste, die ein oder mehrere Stickstoff- und/oder Sauerstoff- und/oder Schwefelatome enthalten, stehen. Weiterhin werden 1,3-Dioxane der allgemeinen Formel (II) bevorzugt, in der R³ ein geradkettiger oder verzweigter Alkylrest mit 1 bis 12, insbesondere 1 bis 8, vorzugsweise 1 bis 4 Kohlenstoffatomen ist. Besonders bevorzugt steht R³ für Wasserstoff.

Beispiele für Alkyl-, Alkenyl- oder Alkinylreste sind der Methyl-, Ethyl-, n-Propyl-, i-Propyl-, Propenyl-, i-Propenyl-, n-Butyl-, i-Butyl, n-Butenyl, i-Butenyl, n-Butinyl-, Pentyl-, Pentenyl-, Pentinyl-, Hexyl-, Hexenyl-, Heptyl-, Heptenyl-, Octyl-, Octenyl-, Nonyl-, Nonenyl-, Decyl-, Decenyl-, Dodecyl- oder Dodecenylrest.

Cycloalkylreste werden beispielhaft durch den Cyclopentyl-, Cyclohexyl-, Cycloheptyl-, Cyclopentenyl- oder Cyclohexenylrest vertreten.

Als aromatische Reste kommen z.B. der Phenyl-, Benzyl-, 2-Phenylethyl-, 2-Phenylpropyl-, 3-Phenylpropyl-, 2-Phenylbutyl-, 3-Phenylbutyl-, 4-Phenylbutylrest in Betracht.

Beispiele für heterocyclische Reste sind der Furan-, Dihydrofuran-, Tetrahydrofuran-, Thiophen-, Dihydrothiophen-, Pyridin- und Thiopyranrest.

Die Alkyl-, Cycloalkyl-, aromatischen und heterocyclischen Reste können substituiert sein, insbesondere durch Reste, die unter den Reaktionsbedingungen inert sind wie Halogen, Alkoxy-, Carboxy- oder Carboxylatgruppen. Es ist aber in Einzelfällen auch nicht ausgeschlossen, daß bewußt Substituenten ausgewählt werden, die im Verlauf der Reaktion verändert werden, z.B. Carbonylgruppen, die in Hydroxymethylreste übergeführt werden.

Die als Ausgangsmaterial in das erfindungsgemäße Verfahren eingesetzten 1,3-Dioxane sind auf verschiedenen Wegen zugänglich. Ein bewährtes Verfahren ist die säurekatalysierte Addition von 1,3-Diolen an Aldehyde oder Ketone oder die Umacetalisierung von Acetalen bzw. Ketalen, insbesondere solchen, die sich von niedrig siedenden Alkoholen ableiten, mit 1,3-Diolen in Gegenwart von Säuren.

Geeignete Diolkomponenten sind z.B. Propandiol-1,3, 2-Methylpropandiol-1,3, 2-Ethylpropandiol-1,3, 2-Phenylpropandiol-1,3, 2,2-Dimethylpropandiol-1,3 (Neopentyl glycol), 2,2-Diethylpropandiol-1,3, 2-Methyl-2-ethylpropandiol-1,3, 2-Methyl-2-propylpropandiol-1,3, 2-Methyl-2-butylpropandiol-1,3, 2-Methyl-2-phenylpropandiol-1,3, 2-Ethyl-2-butylpropandiol-1,3, 1,1-Dimethylolcyclohexan, 1,1-Dimethylolcyclopentan, 3,3-Dimethyloltetrahydrofuran, 3,3-Dimethyloltetrahydropyran und 2,2,4-Trimethylpentandiol-1,3.

Als Carbonylverbindungen werden mit den 1,3-Diolen z.B. aliphatische, aromatische oder heterocyclische Aldehyde und Ketone oder deren Acetale bzw. Ketale umgesetzt. Die Aldehyde können ebenso wie die Ketone, gesättigt oder ungesättigt sein.

Beispiele für geeignete aliphatische Aldehyde sind Formaldehyd, Acetaldehyd, Propionaldehyd, Butyraldehyd, Isobutyraldehyd, n-Pentanal, 2-Methylbutanal, 3-Methylbutanal, n-Hexanal, 2-Methylpentanal, 3,3-Dimethylbutanal, 2-Ethylhexanal, 2-Methyldecanal, ferner Dialdehyde wie Glyoxal, Methylglyoxal, Malondialdehyd, Succindialdehyd und Glutardialdehyd, sowie substituierte Aldehyde wie 3-Hydroxy-2,2-dimethylpropanal (Hydroxypivalin aldehyd), Methoxypivalinaldehyd, Butoxypivalinaldehyd, 4-Acetoxybutyraldehyd und 5-Formylvalerianaldehyd.

Auch ungesättigte aliphatische Aldehyde können als Reaktionskomponente für die 1,3-Diole verwendet werden, z.B. Acrolein, α-Methylacrolein, α-Ethylacrolein und höhere α-Alkyl-, Isoalkyl- und Alkenylacroleine wie But-2-enal, But-3-enal, 2-Methylbut-2-enal, 2-Methylpent-2-enal, 2-Ethylhex-2-enal, 2,2-Dimethylpent-4-enal, 2-Methyl-4-acetoxy-but-2-enal, 2-Methoxymethylacrolein, 2-(3-Methoxycarbonylpropyl)-acrolein, 2-Methyl-4-chlor-but-2-enal.

Als aromatische Aldehyde seien beispielhaft genannt Benzaldehyd, p-Methoxybenzaldehyd, Phenylacetaldehyd, 2-Phenylpropanal, 3-Phenylpropanal, 2-Hydroxybenzaldehyd, 3-Hydroxy-4-methoxybenzaldehyd, Zimtaldehyd und Benzylacrolein.

Beispiele für heterocyclische Aldehyde sind Tetrahydrofuryl-2-aldehyd, Tetrahydrofuryl-3-aldehyd, Tetrahydrothienyl-2-aldehyd, Tetrahydrothienyl-3-aldehyd, 5,6-Dihydropyranyl-6-aldehyd, 2,5-Dimethyl-5,6-dihydropyranyl-6-aldehyd, Furyl-2-aldehyd, Furyl-3-aldehyd, Thienyl-3-aldehyd und 2-, 3- oder 4-Pyridylaldehyd.

Zur Herstellung der 1,3-Dioxane geeignete Ketone sind z.B. Aceton, Methylethylketon, Diethylketon, Methylisopropylketon, Diisopropylketon, Diisobutylketon, Methylisobutylketon, Methoxyaceton, Methylphenylketon, Methylisopropenylketon, Methylisobutenylketon, Cyclopentanon, Cyclohexanon, Dimethylcyclopentanon, Dimethylcyclohexanone, Cyclohexenon, 3,5,5-Trimethylcyclohexenon-2, Methyl-, Ethyl- und Vinylphenylketon, Methylfurylketon, Acetylaceton und Acetessigester.

Die vorstehend aufgeführten Diole, Aldehyde und Ketone als Bausteine für die erfindungsgemäß eingesetzten 1,3-Dioxane sollen lediglich die Anwendungsbreite des neuen Verfahrens skizzieren, es jedoch nicht auf diese beispielhaft genannten Verbindungen beschränken.

Andere Reaktionen, die zu 1,3-Dioxanen führen, sind die Umsetzung von Grignard-Reagenzien mit Orthoestern sowie die Reaktion von Alkoholaten von Diolen entweder mit geminalen Halogeniden oder mit α-Haloethern. Schließlich sind 1,3-Dioxane auch durch Prinsreaktion, d.h. die Addition eines Olefins an Formaldehyd in Gegenwart einer Säure, zugänglich.

Die Hydrierung der 1,3-Dioxane nach dem erfindungsgemäßen Verfahren erfolgt bei 40 bis 500°C. Bevorzugt werden Temperaturen von 100 bis 450°C und insbesondere von 150 bis 350°C. Der Reaktionsdruck kann über einen weiten Bereich eingestellt werden und liegt bei 0,1 bis 35 MPa, wobei Drücke von 3 bis 15 MPa bevorzugt werden.

Das Molverhältnis von Wasserstoff zu 1,3-Dioxanen beträgt 0,2 : 1 bis 250 : 1 und insbesondere 1 : 1 bis 100 : 1 eingesetzt.

Die Umsetzung der Reaktanten erfolgt in Gegenwart von Hydrierkatalysatoren. Als hydrieraktive Komponente enthalten diese Katalysatoren ein Metall oder mehrere Metalle der Gruppen VIB, VIIIB und IB des Periodensystems der Elemente (entsprechend der von Chemical Abstracts verwendeten Gruppenbezeichnung, die auch weiterhin benutzt wird), ausgenommen Chrom und Nickel. Bevorzugt als hydrieraktive Komponenten werden Molybdän, Wolfram, Ruthenium, Kobalt, Rhodium, Iridium, Palladium, Platin und insbesondere Kupfer.

Neben den speziellen hydrieraktiven Metallen ist für die erfindungsgemäß angewandten Katalysatoren die Auswahl der Träger charakteristisch. Für diesen Zweck werden acide Verbindungen einzeln oder in Form von Gemischen unterschiedlicher acider Substanzen eingesetzt.

Besonders geeignet aus der Vielzahl acider Verbindungen sind Zeolithe. Unter dieser Bezeichnung versteht man kristalline, hydratisierte Alumosilikate mit Gerüststruktur, die Alkali- und/oder Erdalkalikationen enthalten. Sie kommen natürlich vor und werden auch synthetisch hergestellt. Zeolithe zeichnen sich strukturell durch ein regelmäßiges System aus interkristallinen Hohlräumen aus, das über Porenöffnungen für Moleküle gleicher Größenordnung zugänglich ist. Das Gerüst der Zeolithe besteht aus Tetraedern, wobei um das Zentralatom, das entweder ein Si⁴⁺- oder Al³⁺-Kation sein kann, vier Sauerstoffatome angeordnet sind. Jedes in die Struktur eingebaute Aluminiumatom führt zu einer negativen Ladung des Gerüsts, die durch Kationen, wie Alkalimetall- oder Erdalkalimetallionen, ausgeglichen wird. Ein Austausch der Kationen ist möglich: Zeolithe sind anorganische Ionenaustauscher. So können z.B. Alkalimetallionen durch Wasserstoffionen ersetzt werden. Auf diese Weise läßt sich die katalytische Aktivität der Zeolithe variieren, die an das Vorliegen acider Zentren in der interkristallinen Oberfläche gebunden ist. Die Räume zwischen den Tetraedern sind von Wassermolekülen besetzt, eine Dehydratation durch Trocknen oder Calcinieren ist möglich.

Im Kristallgitter synthetisch gewonnener Zeolithe kann Aluminium durch andere Elemente, wie Bor, Gallium, Eisen, Chrom, Vanadium, Arsen, Antimon, isomorph ersetzt werden. Silicium läßt sich durch vierwertige Elemente, wie Germanium, Titan, Zirkon und Hafnium isomorph substituieren.

Art und Umfang des Austausches von Aluminium und/oder Silicium erlauben es, die katalytischen Eigenschaften der Zeolithe gezielt zu beeinflussen und individuellen Erfordernissen anzupassen.

Entsprechend ihrer Struktur unterteilt man die Zeolithe in verschiedene Gruppen. In den Zeolithen der Mordenit-Gruppe bilden die Grundeinheiten der Struktur, die SiO₄- und AlO₄-Tetraeder, Ketten. Die Zeolithe der Chabasit-Gruppe sind aus Tetraederschichten aufgebaut. Bei den Zeolithen der Faujasit-Gruppe sind die Tetraeder zu Polyedern angeordnet, z.B. in Form eines Kubooctaeders. Je nach Verknüpfung der Kubooctaeder entstehen unterschiedlich große Hohlräume und Poren, dementsprechend unterscheidet man z.B. Zeolithe vom Typ A, L, X oder Y.

Als Trägerkomponente für die im erfindungsgemäßen Verfahren eingesetzten Katalysatoren eignen sich Zeolithe aus der Faujasit-Gruppe, z.B. der Zeolith Y, Zeolithe aus der Mordenit-Gruppe oder engporige Zeolithe, z.B. vom Erionit- bzw. Chabasit-Typ. Bevorzugt werden Zeolithe vom Pentasil-Typ eingesetzt, die als Grundbaustein einen aus SiO₄-Tetraedern aufgebauten Fünfring besitzen und ein hohes SiO₂/Al₂O₃-Verhältnis aufweisen. Ihre Porengrößen liegen zwischen denen der Zeolithe vom Typ A (Porenöffnungen 4,1 Å) und denen vom Typ X oder Y (Porenöffnungen 7,4 Å). Die Pentasil-Zeolithe können chemisch unterschiedlich zusammengesetzt sein. Dementsprechend unterscheidet man Alumo-, Boro-, Eisen-, Gallium-, Chrom-, Arsen-, Antimon- und Bismutsilicatzeolithe oder deren Gemische, sowie Alumo-, Boro-, Gallium- und Eisengermanatzeolithe oder deren Gemische.

Vorzugsweise setzt man als Träger für die im erfindungsgemäßen Verfahren benutzten Katalysatoren Alumo-, Boro- und Eisensilikat-Zeolithe des Pentasil-Typs ein. Der Alumosilikat-Zeolith wird z.B. aus einer Aluminiumverbindung, vorzugsweise Al(OH)₃ oder Al₂(SO₄)₃ und einer Siliciumkomponente, vorzugsweise hochdispersem Siliciumdioxid in wäßriger Aminlösung, insbesondere in einer Lösung von 1,6-Hexandiamin, 1,3-Propandiamin oder Triethylentetramin, gegebenenfalls unter Zusatz von Alkalimetall- oder Erdalkalimetallhydroxid bei 100 bis 220°C unter autogenem Druck hergestellt. Ein derartiges Verfahren ist in EP 0 007 081 und EP 0 007 098 beschrieben. Hierzu gehören auch die isotaktischen Zeolithe nach EP 0 034 727 und EP 0 046 504. Je nach Wahl der Einsatzstoffmengen beträgt in den synthetisierten Alumosilikat-Zeolithen das SiO₂/Al₂O₃-Verhältnis 10 bis 40.000 zu 1 (in mol). Nach einer anderen Arbeitsweise erhält man Alumosilikat-Zeolithe durch Umsetzung von Aluminium- und Siliciumkomponente in einem Ether, wie Diethylenglykoldimethylether, in einem Alkohol, wie Methanol oder 1,4-Butandiol oder in Wasser.

Borosilikat-Zeolithe kann man aus einer Borverbindung, z.B. H₃BO₃, und einer Siliciumverbindung, vorzugsweise hochdispersem Siliciumdioxid, in der wäßrigen Lösung eines Amins, insbesondere 1,6-Hexandiamin, 1,3-Propandiamin oder Triethylentetramin, gegebenenfalls unter Zusatz von Alkali- oder Erdalkalihydroxid, bei 90 bis 200°C unter autogenem Druck synthetisieren. Statt in wäßriger Aminlösung kann man in einem Ether, z.B. in Diethylenglykoldimethylether oder in einem Alkohol z.B. in 1,6-Hexandiol, als Lösungsmittel arbeiten (vgl. EP 0 007 081 und EP 0 007 098).

Zur Gewinnung der Eisensilikat-Zeolithe geht man z.B. von einer Eisenverbindung, vorzugsweise Fe₂(SO₄)₃, und einer Siliciumverbindung, vorzugsweise hochdispersem Siliciumdioxid, aus, die in der wäßrigen Lösung eines Amins, insbesondere 1,6-Hexandiamin, gegebenenfalls unter Zusatz von Alkalimetall- oder Erdalkalimetallhydroxid, bei 100 bis 200°C unter autogenem Druck, umgesetzt werden (vgl. EP 0 007 081 und EP 0 007 098).

Die Alumo-, Boro- oder Eisensilikat-Zeolithe werden nach ihrer Herstellung und Isolierung bei 100 bis 160°C, vorzugsweise 110 bis 130°C, getrocknet und darauf bei 450° bis 550°, vorzugsweise 480 bis 520°C, calciniert. Anschließend werden sie unter Zusatz eines Bindemittels, z.B. zu Strängen oder Tabletten geformt. Als Bindemittel eignen sich die verschiedenen Aluminiumoxide, vorzugsweise Boehmit, amorphe Alumosilikate mit einem SiO₂/Al₂O₃-Gewichtsverhältnis von 0,3 : 1 bis 18 : 1, bevorzugt 3 : 1 bis 5 : 1, Siliciumdioxid, insbesondere hochdisperses SiO₂, Gemische aus hochdispersem SiO₂ und hochdispersem Al₂O₃, hochdisperses TiO₂ sowie Ton. Bindemittel und Zeolith werden im Gewichtsverhältnis 90 : 10 bis 40 : 60 verwendet. Nach der Formung werden die Formkörper erneut 10 bis 20 h bei 110 bis 130°C getrocknet und bei 400 bis 550°C zwischen 10 und 20 h calciniert.

Statt unmittelbar nach Herstellung, Isolierung und Trocknung zu calcinieren, können die Zeolithe auch getrocknet und geformt und im Anschluß daran calciniert werden. Schließlich ist es auch möglich, auf die Verwendung von Bindemitteln zu verzichten und die Formgebung unter Einsatz von Formgebungs- oder Peptisierungshilfsmitteln, wie Ethylcellulose, Stearinsäure, Kartoffelstärke, Ameisensäure, Oxalsäure, Essigsäure, Salpetersäure, Ammoniak, Amine, Silikoester oder Graphit oder deren Gemische, vorzunehmen.

Zeolithe, die auf Grund ihrer Herstellung Alkalimetall- oder Erdalkalimetallionen, jedoch keine oder nicht ausreichend viele H⁺-Ionen enthalten, müssen durch Ionenaustausch in die acide, katalytisch wirksame H-Form umgewandelt werden. Hierzu behandelt man sie mit Säuren oder führt Ammoniumionen ein und calciniert anschließend. Durch abgestuften Ionenaustausch kann die für den speziellen Anwendungsfall erforderliche Acidität eingestellt werden.

Weiterhin können die Zeolithe durch Ionenaustausch oder durch Imprägnieren mit bestimmten Metallen modifiziert werden, um z.B. die Selektivität der Umsetzung zu verbessern oder die Katalysatorlebensdauer zu erhöhen. Bewährt hat sich die Dotierung der Zeolithe mit Übergangsmetallen der Gruppen VIB, VIIIB, IB, IIB, ausgenommen Chrom und Nickel, wie Molybdän, Wolfram, Eisen, Kupfer, Zink, mit Edelmetallen, wie Palladium und Platin und mit Metallen der Seltenen Erden, wie Lanthan, Cer und Praseodym.

Zur Dotierung durch Ionenaustausch behandelt man geformten oder nicht geformten Zeolith z.B. bei Temperaturen von 20 bis 100°C mit einer wäßrigen oder ammoniakalischen Lösung eines Salzes, beispielsweise eines Halogenids, Nitrats oder Acetats der vorbeschriebenen Metalle. Der Ionenaustausch kann mit Zeolithen in der Wasserstoff-, Ammonium- oder Alkalimetallform vorgenommen werden.

Beispielsweise legt man Stränge oder Pellets des Zeoliths in der H-Form in einer Kolonne vor und leitet eine ammoniakalische Pd(NO₃)₂-Lösung bei Temperaturen von 30 bis 80°C im Kreislauf während 15 bis 20 h über die Formkörper. Anschließend wird der Zeolith mit Wasser ausgewaschen, bei etwa 150°C getrocknet und bei etwa 550°C calciniert.

Nach einer anderen Variante des Ionenaustauschs suspendiert man pulverförmigen Zeolith in einer Metallsalzlösung, z.B. in einer ammoniakalischen Pd(NO₃)₂-Lösung und rührt bei 40 bis 100°C etwa 24 h. Nach Abfiltrieren, Trocknen bei etwa 150°C und Calcinieren bei etwa 500°C kann der modifizierte Zeolith mit oder ohne Bindemittel zu Strängen, zu Pellets oder zu Wirbelgut weiterverarbeitet werden.

Auch die Dotierung der Zeolithe durch Imprägnieren kann mit Metallsalzen, z.B. Chloriden, Nitraten oder Acetaten in wäßriger, ammoniakalischer oder alkoholischer Lösung erfolgen. Eine mögliche Ausführungsform besteht darin, daß man z.B. Wolframsäure, H₂WO₄, weitgehend in Wasser löst und mit dieser Lösung den geformten oder ungeformten Zeolith über einen bestimmten Zeitraum, z.B. 30 min, tränkt. Darauf entfernt man von der überstehenden Lösung das Wasser durch Verdampfen, trocknet den Zeolith bei etwa 150°C und calciniert bei etwa 550°C. Dieser Tränkvorgang kann mehrfach wiederholt werden, bis der gewünschte Metallgehalt eingestellt ist.

An die Dotierung der Zeolithe mit Metallen, unabhängig davon, ob sie durch Ionenaustausch oder durch Imprägnieren erfolgte, kann sich eine Nachbehandlung mit Wasserstoff anschließen.

Eine weitere Abwandlung der Zeolithe kann in einer Behandlung mit anorganischen oder organischen Säuren, wie Salzsäure, Flußsäure oder Phosphorsäure und/oder mit Wasserdampf bestehen.

Als Träger für Katalysatoren, die im erfindungsgemäßen Verfahren eingesetzt werden, haben sich ferner Phosphate bewährt. Besonders geeignet sind Aluminiumphosphate, Cerphosphate, Zirkonphosphate, Borphosphate, Eisenphosphate, Strontiumphosphate, Siliciumaluminiumphosphate, Siliciumeisenaluminiumphosphate oder deren Gemische.

Träger auf Basis von Aluminiumphosphaten, für die im neuen Verfahren angewandten Katalysatoren, erhält man vorteilhaft durch Synthese unter hydrothermalen Bedingungen. Zu diesen Aluminiumphosphaten gehören z.B. APO-5, APO-9, APO-11, APO-12, APO-14, APO-21, APO-25, APO-31 und APO-33. Diese Aluminiumphosphate besitzen Zeolith-Struktur (vgl. hierzu E.M. Flanigen et al., Structural Synthetic and Physicochemical Concepts in Aluminophosphate-based Molecular Sieves, Innovation in Zeolite Materials Science [Editors: P.J. Grobet et al.] Elsevier, 1988, p. 13 ff).

AlPO₄-5 (APO-5) erhält man beispielsweise durch Umsetzung einer mit Tetrapropylammoniumhydroxid versetzten homogenen Mischung von Orthophosphorsäure und Pseudoboehmit in Wasser in einem Autoklaven bei etwa 150°C und 20 bis 60 h Reaktionszeit unter autogenem Druck. Das abfiltrierte AlPO₄ wird bei 100 bis 160°C getrocknet und bei 450 bis 550°C calciniert.

AlPO₄-9 (APO-9) wird aus Orthophosphorsäure und Pseudoboehmit in einer wäßrigen 1,4-Diazadicyclo-(2,2,2)octan-Lösung bei etwa 200°C und 200 bis 400 h Reaktionszeit unter autogenem Druck synthetisiert.

Die Synthese von AlPO₄-21 (APO-21) erfolgt aus Orthophosphorsäure und Pseudoboehmit in wäßriger Pyrrolidin-Lösung bei 150 bis 200°C und 50 bis 200 h Reaktionszeit unter autogenem Druck.

Als Träger geeignete Aluminiumphosphate können auch durch Fällung erhalten werden. Man gewinnt sie beispielsweise, indem man zu einer Lösung von 92 g Diammoniumhydrogenphosphat in 700 ml Wasser innerhalb 2 h eine Lösung von 268 g Al(NO₃)₃xH₂O in 780 ml Wasser tropft. Durch gleichzeitige Zugabe von 25 %iger NH₃-Lösung hält man einen pH-Wert von 8 ein. Der entstandene Niederschlag wird 12 h nachgerührt, abgesaugt, ausgewaschen und 16 h bei 60°C getrocknet.

Beispiele für Siliciumaluminiumphosphate als Träger für die erfindungsgemäß eingesetzten Katalysatoren sind SAPO-5, SAPO-11, SAPO-31 und SAPO-34. Auch diese Siliciumaluminiumphosphate besitzen Zeolith-Struktur (vgl. hierzu R. Szostak et al., Catalysis Letters 2 (1989), p. 63 ff). Man erhält sie durch Umsetzung einer Mischung aus jeweils einer Silicium-, Aluminium- und Phosphorkomponente in wäßrigen, aminoorganischen Lösungen bei 100 bis 250°C und 2 h bis 2 Wochen Reaktionszeit unter autogenem Druck. SAPO-5 wird z.B. durch Mischen einer Suspension von SiO₂ in einer wäßrigen Tetrapropylammoniumhydroxid-Lösung mit einer Suspension aus Pseudoboehmit und Orthophosphorsäure in Wasser und anschließende Umsetzung bei 150 bis 200°C und 20 bis 200 h Reaktionszeit in einem Rührautoklaven unter autogenem Druck erhalten. Das abfiltrierte Pulver wird bei 110 bis 170°C getrocknet und bei 450 bis 550°C calciniert.

Andere geeignete Siliciumaluminiumphosphate sind z.B. auch ZYT-5, ZYT-6, ZYT-7, ZYT-9, ZYT-11 und ZYT-12.

Borphosphate, die als Träger für die im Verfahren eingesetzten Katalysatoren dienen können, gewinnt man z.B. durch Mischen und Kneten von konzentrierter Borsäure und Phosphorsäure, Trocknen des Gemischs und Calcinieren in Inertgas-, Luft- oder Dampfatmosphäre bei 250 bis 650°C, vorzugsweise 300 bis 500°C.

Auch die Träger auf Phosphatbasis können zur Erhöhung der Selektivität, des Umsatzes und der Lebensdauer modifiziert werden. Ein Weg hierzu ist die Dotierung der nicht geformten oder geformten Phosphate mit Metallsalzen durch Ionenaustausch oder Imprägnieren. Für die Dotierung setzt man Übergangsmetalle der Gruppen IVB bis VIIIB des Periodensystems, ausgenommen Chrom und Nickel, wie Titan, Zirkonium, Vanadium, Niobium, Molybdän, Wolfram, Mangan, Rhenium, Eisen, Ruthenium, Osmium, Kobalt, Rhodium, Iridium, Palladium, Platin, Übergangsmetalle der Gruppen IB und IIB des Periodensystems wie Kupfer, Silber, Zink, ferner Zinn, die Metalle der Seltenen Erden, wie Lanthan, Cer, Praseodym, Neodym, Erbium, Ytterbium und weiterhin Uran ein. Alkalimetalle, wie Lithium, Kalium, Caesium, Erdalkalimetalle, z.B. Magnesium, Calcium, Strontium, Metalle der Gruppen IIIA, IVA und VA des Periodensystems, wie Aluminium, Gallium, Germanium, Zinn, Blei, Bismut können als zusätzliche Promotoren im Trägermaterial bereits vorhanden sein oder eingebracht werden. Wie die Zeolithe können auch die Phosphate durch Behandlung mit anorganischen oder organischen Säuren abgewandelt werden.

Schließlich finden als Träger für Katalysatoren zur Hydrierung von 1,3-Dioxanen Metalloxide Anwendung, die acide oder amphotere Eigenschaften aufweisen. Geeignete Metalloxide sind z.B. die sauer wirkenden Oxide von Metallen der Gruppen IIIA und IVA und der Gruppen IVB bis VIB des Periodenystems, ausgenommen Chrom, insbesondere Siliciumdioxid in Form von Kieselgel und Kieselgur, ferner Titanoxid, Zirkondioxid, die Phosphoroxide, Vandiumpentoxid, Nioboxide, Bortrioxid, Aluminiumoxid, Molybdänoxide, Wolframoxide und weiterhin Eisenoxide, für sich allein oder in Mischung aus zwei oder mehr dieser Verbindungen.

Es hat sich als vorteilhaft erwiesen, die genannten Oxide mit anorganischen oder organischen Säuren zu behandeln. Als anorganische Säuren kommen z.B. HF, HCl, HBr, HJ, H₂SO₄, H₂SO₃, HNO₃, H₃BO₃, die Phosphorsäuren und deren Mischungen in Betracht. Organische Säuren, die sich zur Behandlung der Oxide eignen, sind z.B. Ameisensäure, Essigsäure, Propionsäure und Oxalsäure allein oder in Mischung. Auch Gemische aus anorganischen und organischen Säuren können verwendet werden. Die Einwirkung der Säuren erfolgt auf das geformte oder nicht geformte Material.

SiO₂ (Silica) in Pulverform wird z.B. 1 h bei 80°C mit 1 n Säure behandelt. Danach wäscht man mit Wasser, trocknet 16 h bei 110°C und calciniert 20 h bei 500°C. Nach einer anderen Arbeitsweise behandelt man SiO₂ vor oder nach dem Formen 1 bis 3 h bei 60 bis 80°C mit 3 bis 25 Gew.-%iger, insbesondere 12 bis 20 Gew.-%iger wäßriger Salzsäure, wäscht das SiO₂ anschließend mit Wasser, trocknet und calciniert bei 400 bis 500°C.

Nach einer besonders zweckmäßigen Ausführungsform behandelt man SiO₂ vor der Verformung durch Erhitzen mit 0,001 n bis 2 n, vorzugsweise 0,05 n bis 0,5 n Flußsäure z.B. unter Rückfluß im allgemeinen über einen Zeitraum von 0,5 bis 5 h, vorzugsweise 1 bis 3 h. Das Trägermaterial wird isoliert, ausgewaschen, zweckmäßig bei Temperaturen von 100 bis 160°C getrocknet und bei 450 bis 600°C calciniert. Nach einer anderen bevorzugten Ausführungsform der Säurebehandlung läßt man auf SiO₂ nach der Verformung bei erhöhter Temperatur, z.B. 50 bis 90°C, vorzugsweise 60 bis 80°C während 0,5 bis 5 h, vorzugsweise 1 bis 3 h, 12 bis 20 Gew.-%ige Salzsäure einwirken. Anschließend wäscht man das Material aus, trocknet es bei 100 bis 160°C und calciniert bei 450 bis 600°C. Der Behandlung mit Flußsäure kann sich auch eine Behandlung mit Salzsäure anschließen.

Phosphorsäure bringt man auf das Metalloxid-Trägermaterial z.B. SiO₂, Al₂O₃ oder TiO₂ durch Tränken oder Versprühen auf. So erhält man einen phosphorsäurehaltigen Träger beispielsweise durch Behandlung von SiO₂ mit H₃PO₄- oder NaH₂PO₄-Lösung und anschließendes Trocknen bzw. Calcinieren. Phosphorsäure kann auch zusammen mit Kieselgel in einem Sprühturm versprüht werden. Diesem Vorgang schließt sich eine Trocknung und meist eine Calcinierung an. Schließlich kann man Phosphorsäure in einer Imprägniermühle auf Siliciumdioxid aufsprühen.

Die vorstehend beschriebenen Trägermaterialien vom Zeolith-, Phosphat- und Metalloxidtyp sind Basis für die im erfindungsgemäßen Prozeß eingesetzten Katalysatoren. Hierzu müssen die Träger mit dem oder den hydrieraktiven Komponenten beladen werden. Wie bereits gesagt wurde, handelt es sich bei den hydrieraktiven Komponenten um Metalle der Gruppen VIB, VIIIB, IB und IIB des Periodensystems der Elemente, ausgenommen Chrom und Nickel.

Die Vereinigung von Träger und hydrieraktivem Metall kann auf verschiedenen Wegen erfolgen. Sind die Träger zum Ionenaustausch befähigt, dann behandelt man sie mit Lösungen der hydrieraktiven Metalle und ersetzt die austauschfähigen Kationen in der Kristallstruktur durch die Ionen der katalytisch wirksamen Metalle. Zweckmäßig setzt man Metallverbindungen ein, deren Anionen thermisch unbeständig sind und durch Erhitzen entfernt werden können, wie Acetat, Nitrat, Carbonat, Oxalat. Der Umfang des Ionenaustausches wird durch die Ionenaustauschisotherme bestimmt. Die Beladung der Träger mit dem aktiven Metall kann auch mit der Dotierung, wie sie vorstehend beschrieben wurde, verbunden werden, indem man für den Ionenaustausch Lösungen vorsieht, die sowohl Ionen des Hydrierals auch des Dotiermetalls enthalten.

In der Praxis führt man den Ionenaustausch in der Weise durch, daß man pulverförmige Molekularsiebe 1 bis 48 h, vorzugsweise 6 bis 36 h bei 20 bis 80°C mit einer ammoniakalischen Metallsalzlösung rührt. Aus der Differenz der Metallionenkonzentration vor und nach dem Ionenaustausch ergibt sich die Metallkonzentration auf dem Träger. Das mit dem Metall beladene Pulver wird mit destilliertem Wasser gewaschen, bei 110 bis 160° getrocknet und bei 400 bis 650°C calciniert unter Einhaltung einer Aufheizgeschindigkeit von 0,1 bis 10°C min⁻¹.

Ein anderes Verfahren zum Aufbringen der Hydrierkomponente besteht in der Tränkung des Katalysatorträgers mit der Metallsalzlösung. In der Praxis- rührt man z.B. den Katalysatorträger bei 20 bis 80°C 1 bis 48 h, vorzugsweise 6 bis 36 h mit einer ammoniakalischen Lösung des Metallsalzes. Das Lösungsmittel wird abdestilliert, der beladene Träger bei 110 bis 160°C getrocknet und bei 400 bis 650°C calciniert. Auch im Falle des Tränkverfahrens können Aufbringen der hydrieraktiven Komponente und Dotierung des Trägers in einem Schritt durchgeführt werden.

Eine weitere Variante zur Herstellung von Hydrierkatalysatoren, die für das neue Verfahren geeignet sind, ist die gemeinsame Fällung von Hydrier- und Trägerkomponente. Hierbei sind zwei Möglichkeiten zu unterscheiden. Entweder wird die hydrieraktive Komponente auf den vorgefertigten Träger gefällt oder man fällt hydrieraktive Komponente und Träger gemeinsam aus. In der Praxis legt man z.B. die Metallsalzlösung, in der das Trägermaterial suspendiert ist, vor und fällt mit einem basischen Reagenz das Metall als schwerlösliche Verbindung, beispielsweise als Hydroxid, Hydrogencarbonat, Carbonat oder basisches Carbonat, auf den Träger aus. Bei der gleichzeitigen Fällung von Hydrier- und Trägerkomponente wird eine gemeinsame Lösung der Ausgangsverbindungen simultan mit dem Fällungsmittel umgesetzt. Das Fällungsgut wird gegebenenfalls bei Raum- oder erhöhter Temperatur nachgerührt, filtriert, gewaschen, getrocknet und calciniert.

Je nach dem angewandten Verfahren wird das Trägermaterial mit unterschiedlichen Mengen der hydrieraktiven Komponente beladen. Bei der Gewinnung von Katalysatoren durch Ionenaustausch ist die maximale Metallkonzentration durch das Austauschvermögen des Trägermaterials begrenzt. Üblicherweise enthalten derartige Katalysatoren 0,5 bis 15 Gew.-% der hydrieraktiven Komponente, bezogen auf den Katalysator.

Bei Tränkungsverfahren kann man den Beladungsgrad des Trägers durch Variation der Konzentration der Metallsalzlösung und durch ein- oder mehrfache Wiederholung des Tränkungsvorganges über weite Bereiche variieren. Metallkonzentrationen von 0,1 bis 30, vorzugsweise 0,5 bis 10 und insbesondere 1 bis 5 Gew.-%, bezogen auf den Katalysator, können eingestellt werden.

Die größte Flexibilität hinsichtlich der Einstellung des Metallgehaltes im Katalysator erzielt man durch Anwendung von Fällungsverfahren, gleichgültig, ob die Metallkomponente auf den vorgefertigten Träger aufgebracht wird oder Metallkomponente und Trägerkomponente gemeinsam ausgefällt werden. Der gewünschte Metallgehalt kann bei dieser Arbeitsweise durch Wahl der Mengenverhältnisse von Metall und Träger frei bestimmt werden. Üblicherweise enthalten Fällungskatalysatoren, in Abhängigkeit vom ausgewählten Metall, 0,1 bis 30, vorzugsweise 0,5 bis 10 und insbesondere 1 bis 5 Gew.-% hydrieraktives Metall, bezogen auf den Katalysator.

Pulverförmige Katalysatoren können nach Isolierung, Trocknung und Calcinierung mit einem Bindemittel zu Strängen oder Tabletten geformt werden. Als Bindemittel eignen sich die verschiedenen Aluminiumoxide, vorzugsweise Boehmit, amorphe Alumosilikate mit einem SiO₂/Al₂O₃-Gew.-Verhältnis von 25 : 75 bis 90 : 5, bevorzugt 75 : 25, Siliciumdioxid, bevorzugt hochdisperses SiO₂, Gemische aus hochdispersem SiO₂ und hochdispersem Al₂O₃, TiO₂, ZrO₂ sowie Ton. Nach der Formung werden die Extrudate oder Preßlinge nochmals getrocknet und gegebenenfalls anschließend calciniert.

Statt sie mit einem Bindemittel zu versehen, können die pulverförmigen Katalysatoren auch unmittelbar nach dem Trocknen zu Tabletten oder Strängen geformt und darauf calciniert werden. Es hat sich bewährt, dem Katalysatorpulver Hilfsmittel zum Strangpressen oder Peptisieren zuzusetzen, z.B. Methylcellulose, Ethylcellulose, Stearinsäure, Kartoffelstärke, Ameisensäure, Essigsäure, Oxalsäure, Alkalilauge, Ammoniak, Amine oder Graphit.

Strang- und Tablettengröße richten sich nach den individuellen Erfordernissen. Üblicherweise setzt man die Katalysatoren als Stränge von 2 bis 4 mm, als Tabletten mit 3 bis 5 mm Durchmesser, als Pellets mit einer Kornfraktion von 1,0 bis 1,6 mm oder pulverförmig, z.B. auch als Wirbelgut mit Korngrößen zwischen 50 und 400 µm ein.

Das erfindungsgemäße Verfahren kann diskontinuierlich oder kontinuierlich betrieben werden. Die diskontinuierliche Reaktionsführung erfolgt in Autoklaven oder Druckrohren. Die kontinuierliche Betriebsweise kann in Festbett- oder Fließbettreaktoren erfolgen. Als Festbettreaktoren gelangen z.B. Schlaufenreaktoren, Hordenreaktoren, Kreisgasreaktoren und vorzugsweise Rohrreaktoren zum Einsatz. Zweckmäßigerweise beträgt bei Rohrreaktoren das Verhältnis von Reaktordurchmesser zu Katalysatorpellets 2 : 1 bis 20 : 1 und insbesondere 4 : 1 bis 10 : 1.

In den folgenden Beispielen wird das erfindungsgemäße Verfahren erläutert, jedoch nicht auf die beschriebenen Ausführungsformen beschränkt.

### Durchführung der Versuche

Die Beispiele 1 bis 4 werden unter isothermen Bedingungen in einem 75 ml-Rührautoklaven durchgeführt. 5,5-Dimethyl-2-phenyl-1,3-dioxan wird-als Lösung in Dioxan nach vorherigem Trocknen in den Reaktor eingeführt. Zu dem Ausgangsstoff werden 2 g Katalysator gegeben, der zuvor 30 min auf 300°C erhitzt worden war. Die Apparatur wird mit Stickstoff gespült und darauf bis zum Erreichen des Reaktionsdruckes mit Wasserstoff aufgefüllt.

Für die Beispiele 5 bis 9 wird ein Rührautoklav mit einem Gesamtvolumen von 350 ml verwendet. Es gelangen 200 ml einer Lösung von 5,5-Dimethyl-2-phenyl-1,3-dioxan in Cyclohexan (entsprechend 20 g cyclisches Acetal) sowie 1 g pulverförmiger Katalysator zur Anwendung.

Für die kontinuierlichen Versuche (Beispiele 10 bis 14) wird ein Rohrwendelreaktor von 900 mm Länge und 6 mm Innendurchmesser eingesetzt. Im Reaktor werden 2 g Katalysator angeordnet, der 30 min im Stickstoffstrom (4,5 l/h) bei Reaktionstemperatur getrocknet und darauf durch Überleiten eines Wasserstoffstroms (10 l/h) 12 h bei 250 bis 400°C reduziert wird. Anschließend wird die Lösung der Ausgangsstoffe mit Hilfe einer Pumpe in den Reaktor gefördert. Der Produktstrom wird in einer Kühlfalle bei - 30°C kondensiert, auf Raumtemperatur erwärmt und gaschromatographisch untersucht.

Die Versuche 13 bis 15 werden in einem Durchfluß-Hydrierreaktor von 19 mm Innendurchmesser durchgeführt. Über 15 ml Katalysator, der zuvor 3 h mit 15 l Wasserstoff je h unter Normaldruck bei 200°C reduziert wurde, werden 60 ml einer Lösung, die je l 86,4 g 5,5-Dimethyl-2-phenyl-1,3-dioxan in Cyclohexan enthält, bei 160 bis 180°C geleitet. Die analytische Untersuchung des Produktes erfolgt wie vorstehend beschrieben.

### Katalysatorträger

### 1. H-[B]-silicalit (H-[B]-ZSM5)

Der Borozeolith des Pentasiltyps wird durch hydrothermale Synthese hergestellt. Hierzu setzt man 640 g hochdisperses SiO₂, 122 g H₃BO₃ und 8000 g einer wäßrigen 1,6-Hexandiamin-Lösung (Amin : Wasser = 1 : 1 in Gew.-teilen) bei 170°C unter autogenem Druck in einem Rührautoklav um. Das kristalline Reaktionsprodukt wird abfiltriert, mit Wasser gewaschen, 24 h bei 100°C getrocknet und 24 h bei 500°C calciniert. Der Borozeolith enthält 94,2 Gew.-% SiO₂ und 2,3 Gew.-% B₂O₃. Er wird in Mischung mit Boehmit (60 Gew.-teile Zeolith, 40 Gew.-teile Boehmit) zu Strängen geformt, die 16 h bei 110°C getrocknet und 24 h bei 500°C calciniert werden.
Nachfolgend wird der Träger mit A bezeichnet.

### 2. H-[Al]-ZSM5

Der als Träger verwendete Zeolith mit MFI-Struktur ist ein Handelsprodukt (Produktbezeichnung: SH-55) der Vereinigten Aluminium Werke VAW GmbH. Das SiO₂/Al₂O₃-Molverhältnis beträgt 54 : 1.
Im folgenden wird der Träger mit B bezeichnet.

### 3. Silicalit

Silicalit wird nach folgender Vorschrift hergestellt:
Eine Mischung aus 200 g einer wäßrigen, 1-molaren Lösung von Tetra(n-propyl)ammoniumhydroxid und 200 g Wasser wird unter Rühren zu 78,5 g Tetraethylorthosilicat getropft. Man rührt 1 h nach und fügt 300 g Ethanol hinzu. Diese Mischung wird weitere 2 h gerührt und darauf in einen 2,5 l-Edelstahlautoklaven gegeben. Die hydrothermale Synthese wird unter Rühren (100 Umdrehungen/min) bei 105°C und 96 h Reaktionszeit durchgeführt. Die entstandenen Kristalle werden in einer Zentrifuge abgetrennt, dreimal mit Wasser gewaschen, darauf 16 h bei 110°C getrockent und 12 h an der Luft bei 550°C calciniert (Aufheizgeschwindigkeit: 1°C/min).
Nachfolgend wird der Träger mit C bezeichnet.

### 4. Al₂O₃

Das als Träger eingesetzte Aluminiumoxid wird von der BASF hergestellt und unter der Bezeichnung D10-10 in den Handel gebracht. Es enthält jeweils weniger als 0,1 Gew.-% Na, K und Fe. Seine innere Oberfläche beträgt etwa 230 m²/g, das Porenvolumen (bestimmt durch Wasseraufnahme) etwa 0,7 cm³/g und das Litergewicht etwa 650 g/l. Das Aluminiumoxid ist bis 500°C beständig.
Nachfolgend wird der Träger mit D bezeichnet.

### Katalysatorherstellung

Zur Herstellung der Katalysatoren werden die in Tabelle 2 angegebenen Trägermaterialien verwendet. Zum Aufbringen des hydrieraktiven Metalls pumpt man während 24 h bei 70 bis 80°C eine Lösung von Cu(NO₃)₂ · 3H₂O oder Cu(CH₃COO)₂ · H₂O über den Träger. An Stelle einer wäßrigen kann man auch eine ammoniakalische Lösung des Kupfersalzes verwenden. Die Metallbeladung erfolgt dann bei einem pH-Wert von 10,5. Bei dieser Arbeitsweise wird in kürzerer Zeit eine höhere Kupferkonzentration auf dem Träger erzielt als bei Einsatz wäßriger Lösungen.

Statt die Kupfersalzlösung über das Trägermaterial zu pumpen, kann man Kupfersalzlösung und Träger auch in einem Kolben bei Raumtemperatur rühren.

Zur Beladung durch Ionenaustausch wird der Träger zwei- oder dreimal mit der wäßrigen Metallsalzlösung während 24 h gerührt. Das Metallsalz gelangt in großem Überschuß zur Anwendung. Nach Beendigung des Ionenaustausches filtriert und wäscht man den Katalysator und trocknet und calciniert ihn anschließend.

In Tabelle 1 sind die in den Versuchen eingesetzten Katalysatoren zusammengestellt. Die Art der Träger ist, wie oben beschrieben, durch die Buchstaben A, B, C und D bezeichnet. Unterschiedlicher Kupfergehalt bei gleichem Träger wird durch nachgetellte Ziffern wiedergegeben.

**Tabelle 1**

| Katalysatoren | | |
|---|---|---|
| Bezeichnung | Träger | Cu-Konzentration [Gew.-%, bez. auf Katalysator] |
| A-1 | H-[B]-silicalit | 4,0 |
| A-2 | H-[B]-silicalit | 7,0 |
| B | H-[A1]-ZSM5 | 3,8 |
| C-1 | Silicalit | 5,9 |
| C-2 | Silicalit | 12,3 |
| D-1 | Al₂O₃ | 7,0 |
| D-2 | Al₂O₃ | 7,6 |
| D-3 | Al₂O₃ | 8,3 |

**Tabelle 2**

| Reduktion von 5,5-Dimethyl-2-phenyl-1,3-dioxan zu 3-Benzyloxy-2,2-dimethylpropanol | | | | | | | |
|---|---|---|---|---|---|---|---|
| Beispiel | Reaktor | Katalysator | Temp. [°C] | Druck [MPa] | Dioxan-Umsatz [%] | Selektivität bzgl. Alkohol [%] | Anmerkung |
| 1 | Autoklav | C-1 | 190 | 9 | 5,0 | 42,0 | |
| 2 | Autoklav | C-2 | 190 | 9 | 50,0 | 75,0 | |
| 3 | Autoklav | A-1 | 190 | 9 | 70,0 | 90,0 | |
| 4 | Autoklav | B | 190 | 9 | 98,0 | 85,0 | |
| 5 | Autoklav | A-3 | 195 | 9 | 89,4 | 97,0 | |
| 6 | Autoklav | A-3 | 195 | 9 | 69,4 | 82,0 | |
| 7 | Autoklav | D-1 | 195 | 9 | 64,8 | 82,2 | |
| 8 | Autoklav | D-3 | 195 | 9 | 80,6 | 87,5 | |
| 9 | Autoklav | D-3 | 205 | 9 | 93,6 | 92,0 | |
| 10 | Rohr | C-2 | 190 | 9 | 50,0 | 92,0 | |
| 11 | Rohr | A-2 | 300 | 0,1 | 74,0 | 44,1 | |
| 12 | Rohr | A-2 | 190 | 0,1 | 40,0 | 74,0 | |
| 13 | Rohr | D-2 | 180 | 3 | 98,1 | 59,7 | |
| 14 | Rohr | D-2 | 160 | 3 | 91,3 | 76,7 | |
| 15 | Rohr | D-2 | 170 | 3 | 99,2 | 64,6 | |

## Patentansprüche

1. Verfahren zur Herstellung von 3-Oxyalkyl-propan-1-olen der allgemeinen Formel 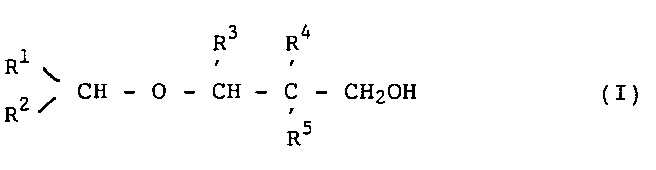 indem man [1,3]Dioxane der allgemeinen Formel 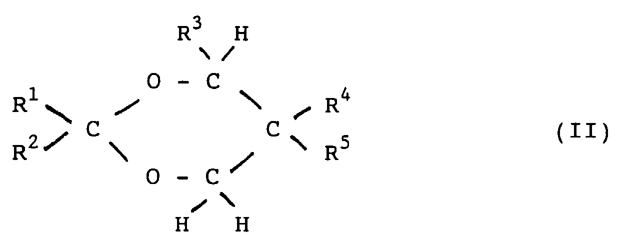 in der R^{1,} R², R⁴ und R⁵ gleich oder verschieden sind und Wasserstoff, geradkettige oder verzweigte Alkyl-, Alkenyl- oder Alkinylreste mit bis zu 18 Kohlenstoffatomen, Cycloalkyl- oder Cycloalkenylreste mit 5 bis 8 Kohlenstoffatomen, Aryl-, Alkylaryl-, Aralkyl-, Aralkenyl- oder Alkenylarylreste mit 6 bis 16 Kohlenstoffatomen oder heterocyclische Reste bedeuten, wobei jeweils die Reste R¹ und R² und/oder R⁴ und R⁵ zusammen mit dem Kohlenstoffatom, an das sie gebunden sind, ein Cycloalkan, Cycloalken oder einen Heterocyclus mit 5 bis 7 Ringgliedern bilden können, R¹, R², R⁴ und R⁵ überdies Substituenten, insbesondere solche, die unter den Reaktionsbedingungen inert sind, tragen können und R³ für Wasserstoff oder einen geradkettigen oder verzweigten Alkylrest steht, bei Drücken von 0,1 bis 35 MPa und Temperaturen von 40 bis 500°C in Gegenwart von Katalysatoren hydriert, dadurch gekennzeichnet, daß die Katalysatoren als hydrieraktive Komponente ein Metall oder mehrere Metalle der Gruppen VIB, IIIB und IB des Periodensystems der Elemente, ausgenommen Chrom und Nickel, und einen aciden Träger enthalten.

2. Verfahren nach Anspruch 1, dadurch gekennzeichnet, daß als 1,3-Dioxane Verbindungen der allgemeinen Formel (II) 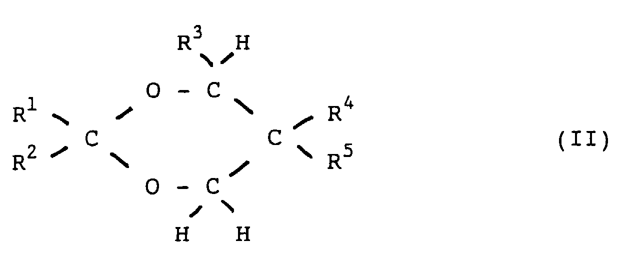 umgesetzt werden, in der R¹, R², R⁴ und R⁵ gleich oder verschieden sind und Wasserstoff, geradkettige oder verzweigte Alkylreste mit 1 bis 12 und insbesondere 1 bis 6 Kohlenstoffatomen, Alkenyl- oder Alkinylreste mit 2 bis 12 und insbesondere 2 bis 6 Kohlenstoffatomen, Cycloalkyl- oder Cycloalkenylreste mit 5 oder 6 Kohlenstoffatomen, Aryl-, Alkylaryl-, Aralkyl-, Aralkenyl- oder Alkenylarylreste mit 6 bis 12 Kohlenstoffatomen, heterocyclische Reste, die ein oder mehrere Stickstoff- und/oder Sauerstoff- und/oder Schwefelatome enthalten, bedeuten und R³ ein geradkettiger oder verzweigter Alkylrest mit 1 bis 12, insbesondere 1 bis 8, vorzugsweise 1 bis 4 Kohlenstoffatomen und besonders bevorzugt Wasserstoff ist.

3. Verfahren nach Anspruch 1 oder 2, dadurch gekennzeichnet, daß die hydrieraktive Komponente Molybdän, Wolfram, Ruthenium, Kobalt, Rhodium, Iridium, Palladium, Platin und insbesondere Kupfer ist.

4. Verfahren nach einem oder mehreren der Ansprüche 1 bis 3, dadurch gekennzeichnet, daß die Katalysatoren 0,5 bis 30 Gew.-% der hydrieraktiven Komponente (bezogen auf den Katalysator) enthalten.

5. Verfahren nach einem oder mehreren der Ansprüche 1 bis 4, dadurch gekennzeichnet, daß die Katalysatoren als Träger Zeolithe enthalten.

6. Verfahren nach Anspruch 5, dadurch gekennzeichnet, daß der Träger ein Zeolith des Pentasil-Typs ist.

7. Verfahren nach Anspruch 5 oder 6, dadurch gekennzeichnet, daß der Zeolith ein Alumo-, Boro- oder Eisensilikatzeolith des Pentasil-Typs ist.

8. Verfahren nach einem oder mehreren der Ansprüche 5 bis 7, dadurch gekennzeichnet, daß der Zeolith mit Übergangsmetallen der Gruppen VIB, VIIIB, IB, IIB des Periodensystems, ausgenommen Chrom und Nickel, dotiert ist.

9. Verfahren nach einem oder mehreren der Ansprüche 5 bis 8, dadurch gekennzeichnet, daß der Zeolith mit Edelmetallen dotiert ist.

10. Verfahren nach einem oder mehreren der Ansprüche 5 bis 6, dadurch gekennzeichnet, daß der Zeolith mit Metallen der Seltenen Erden dotiert ist.

11. Verfahren nach einem oder mehreren der Ansprüche 1 bis 4, dadurch gekennzeichnet, daß der Hydrierkatalysator als Träger Phosphate enthält.

12. Verfahren nach Anspruch 11, dadurch gekennzeichnet, daß die Phosphate Aluminiumphosphat, Cerphosphat, Zirkonphosphat, Borphosphat, Eisenphosphat, Strontiumphosphat, Siliciumaluminiumphosphat, Siliciumeisenaluminiumphosphat sind und einzeln oder als Gemisch aus zwei oder mehr Phosphaten eingesetzt werden.

13. Verfahren nach Anspruch 11 oder 12, dadurch gekennzeichnet, daß die Phosphate mit Metallen der Gruppen IA, IIA, IIIA, IVA oder VA des Periodensystems, mit Übergangsmetallen der Gruppen IVB bis VIIIB, IB oder IIB, ausgenommen Chrom und Nickel, mit Metallen der Seltenen Erden oder mit Uran dotiert sind.

14. Verfahren nach einem oder mehreren der Ansprüche 1 bis 4, dadurch gekennzeichnet, daß der Hydrierkatalysator als Träger ein acides oder amphoteres Metalloxid enthält.

15. Verfahren nach Anspruch 14, dadurch gekennzeichnet, daß der Träger ein Oxid von Metallen der Gruppen IIIA, IVA oder der Gruppen IVB bis VIB des Periodensystems, ausgenommen Chromoxide, oder ein Gemisch aus zwei oder mehr solcher Oxide ist.

16. Verfahren nach einem oder mehreren der Ansprüche 5 bis 15, dadurch gekennzeichnet, daß die Zeolithe Phosphate und/oder Metalloxide mit anorganischen oder organischen Säuren behandelt sind.

17. Verfahren nach einem oder mehreren der Ansprüche 1 bis 16, dadurch gekennzeichnet, daß die Umsetzung bei Drücken von 3 bis 15 MPa und bei Temperaturen von 100 bis 450°C, vorzugsweise 150 bis 300°C erfolgt.

18. Verfahren nach einem oder mehreren der Ansprüche 1 bis 17, dadurch gekennzeichnet, daß das Molverhältnis von Wasserstoff zu 1,3-Dioxanen 0,2 : 1 bis 250 : 1, insbesondere 1 : 1 bis 100 : 1, beträgt.
